# EUROPÄISCHE PATENTSCHRIFT

(11) **EP 3 446 717 B1**
(45) Veröffentlichungstag und Bekanntmachung des Hinweises auf die Patenterteilung: **29.06.2022**
(21) Anmeldenummer: 17188023.0
(22) Anmeldetag: 25.08.2017
(51) Int. Cl.: A61L 2/04, A61L 2/24, A61L 2/28, A61L 2/18

(54) **VORRICHTUNG ZUR REINIGUNG UND DESINFEKTION VON SPÜLGUT**
DEVICE FOR CLEANING AND DISINFECTING WASH WARE
DISPOSITIF DE NETTOYAGE ET DE DÉSINFECTION DE PRODUIT À LAVER

(43) Veröffentlichungstag der Anmeldung: 27.02.2019
(73) Patentinhaber: Lischka GmbH, 12681 Berlin (DE)
(72) Erfinder: LISCHKA, Leonhard, 16356 Ahrensfelde (DE); KÖLBL, René, 15366 Dahlwitz-Hoppegarten (DE)
(74) Vertreter: Dr. Klemens Schubert Patentanwalt

(56) Entgegenhaltungen:
- EP-A1- 2 241 240
- WO-A1-2015/091750
- DE-A1-102007 005 834
- DE-A1-102007 021 245
- DE-A1-102013 220 035
- PR EN ISO 15883-1: "Reinigungs-/Desinfektionsgeräte", ENTWURF NORM, OESTERREICHISCHES NORMUNGSINSTITUT, WIEN, AT, 1 January 2000 (2000-01-01), page complete, XP002389561,

## Beschreibung

Die vorliegende Erfindung betrifft eine Vorrichtung zur Desinfektion und Reinigung von Spülgut.

Vorrichtungen, die ihre Anwendung in Laboren, Krankenhäusern und Pflegeheimen zur Desinfektion oder Aufbereitung medizinischen Spülgutes, wie Pflegegeschirr, finden, müssen gemäß den gesetzlichen Erfordernissen in regelmäßigen Zeitintervallen auf das Erreichen der notwendigen Desinfektionstemperatur hin kontrolliert werden. Des Weiteren kommt es in diesen Einrichtungen auf eine zügige Aufbereitung an, wobei Spülgut unterschiedlichster Art gereinigt und desinfiziert werden soll.

Bei Desinfektionsverfahren mit feuchter Hitze kann erwartet werden, dass eine Temperatur über eine bestimmte Zeitdauer eine voraussagbare Abtötung von Mikroorganismen, die einer bestimmten Resistenz entsprechen, bewirkt. Wenn besonders resistente Mikroorganismen gewählt werden und eine Anzahl, die die in der Praxis auftretende Menge übersteigt, ist es möglich, die erforderlichen Temperaturen und Einwirkzeiten in einer Norm festzulegen.

In der DE 10 2007 005 834 A1 wird eine Spülmaschine für den gewerblichen Einsatz vorgeschlagen, welche ausgestaltet ist, um im zyklischen Betrieb Spülgut in einer Spülkammer zu reinigen. Die Spülmaschine weist mindestens einen Tank zur Aufnahme einer Spülflüssigkeit sowie mindestens eine Heizvorrichtung zum Aufheizen der Spülflüssigkeit und mindestens einen Temperatursensor zur Erfassung der Temperatur der Spülflüssigkeit auf. In der EP 2 241 240 A1 wird ein Verfahren zur Reinigung eines Reinigungsguts vorgeschlagen. Das Reinigungsgut wird mit mindestens einem Reinigungsfluid beaufschlagt, wobei Wärmeäquivalente erfasst werden, mit denen das Reinigungsgut beaufschlagt wird. Die WO 2015/091750 A1 offenbart eine Reinigungsvorrichtung zur Reinigung von Reinigungsgut. Die Reinigungsvorrichtung umfasst mindestens eine Reinigungskammer und mindestens eine Düse zur Beaufschlagung des Reinigungsguts mit mindestens einem Reinigungsfluid innerhalb der Reinigungskammer. Weiterhin umfasst die Reinigungsvorrichtung mindestens einen Temperatursensor, welcher in mindestens einem Soll-Strahlbereich der Düse angeordnet ist. In der DE 10 2013 220 035 A1 wird ein Verfahren zur Kalibrierung einer Reinigungsvorrichtung vorgeschlagen. Die Reinigungsvorrichtung weist mindestens einen Sensor zur Erfassung mindestens einer Zustandsvariablen auf. Ferner beschreibt die DE 10 2007 021 245 A1 ein Verfahren zum Betrieb einer Reinigungsvorrichtung oder eines Reinigungssystems. Dabei ist die Reinigungsvorrichtung eingerichtet, um einen Reinigungsprozess eines Objektes mit keimtötender Wirkung durchzuführen.

In Deutschland wird gemäß der Norm EN DIN ISO 15883-1 eine Validierung von Reinigungs- und Desinfektionsgeräten mittels thermoelektrischer Verfahren mit Temperaturmessfühlern beschrieben. Diese Validierung dient der routinemäßigen Überprüfung der Desinfektionswirkung in Reinigungs- und Desinfektionsgeräten mit thermischem Desinfektionsschritt.

In der Norm EN DIN ISO 15883-1 wurde der Begriff Ao-Wert als ein Maßstab für die Abtötung von Mikroorganismen in Verfahren mit feuchter Hitze eingeführt. A ist dabei als das Zeitäquivalent in Sekunden bei 80°C definiert, bei dem eine gegebene Desinfektionswirkung erreicht wird. Der D-Wert ist die Zeit, die bei einer bestimmten Temperatur notwendig ist, um die Keimzahl auf 10% zu senken (um 1 log10 -Stufe). Der z-Wert ist die Temperaturerhöhung, die notwendig ist, um den D-Wert auf 1/10 zu reduzieren. Wenn die festgelegte Temperatur 80°C beträgt und der z-Wert 10 ist, wird hierfür der Begriff Ao-Wert verwendet.

Der Ao-Wert eines Desinfektionsverfahrens mit feuchter Hitze ist die Abtötung, angegeben als Zeitäquivalent in Sekunden, bei einer durch das Verfahren an das Produkt übertragene Temperatur von 80°C, bezogen auf Mikroorganismen bei denen der z-Wert 10 beträgt, was bei vielen Mikroorganismen der Fall ist.

Für Desinfektionsverfahren, die Bakterien inklusive Mykobakterien, Pilze und thermolabile Viren umfassen, wird ein Ao-Wert von 600 festgelegt, entsprechend einer Haltezeit von 600 s = 10 min bei 80°C. Die Haltezeit des Ao-Wertes von 600 kann auch auf andere Temperaturen entsprechend umgerechnet werden.

In Bezug auf eine Wirksamkeit gegen thermoresistente Viren sollte ein Ao-Wert von 3000, entsprechend einer Temperatur von 90°C mit einer Haltezeit von 5 min, gewählt werden.

Bei Einhaltung der Werte dieser Norm kann dann davon ausgegangen werden, dass das maschinelle Aufbereitungsverfahren die geforderte Abtötung von Mikroorganismen gewährleistet. Da die Temperatur über das Wasser mittels Beaufschlagung oder Durchströmung auf die Spülgüter übertragen wird, muss gewährleistet sein, dass alle äußeren und inneren Oberflächen erreicht werden.

Im Stand der Technik und gemäß der Norm EN DIN ISO 15883-1 erfolgen daher die Temperaturmessungen im Bereich zwischen den Spülgütern.

Nachteilig an den Vorrichtungen im Stand der Technik ist es, dass der Temperaturfühler innerhalb der Waschkammer angeordnet ist. Demnach ist eine Sicherstellung, dass in allen Bereichen in der Waschkammer die gewünschte Temperatur zur Desinfektion erreicht wird, nicht gewährleistbar. Somit liegt der Ao-Wert am Spülgut unter dem Ao-Wert der Spülkammer und es wird nicht die volle Desinfektionswirkung bzw. Hygienewirkung auf dem Spülgut erreicht.

Aufgabe der vorliegenden Erfindung ist es daher, die Nachteile des Standes der Technik zu überwinden und eine Vorrichtung bereitzustellen, bei der die volle Hygienewirkung auf dem Spülgut erreicht wird.

Eine weitere Aufgabe der vorliegenden Erfindung ist es, ein Verfahren bereitzustellen, welches die Prozesssicherheit hinsichtlich der Sicherstellung und dauerhaften Gewährleistung der thermischen Hygienewirkung innerhalb der Vorrichtung erlaubt.

Die Aufgabe der vorliegenden Erfindung wird durch die Bereitstellung einer Vorrichtung zur Desinfektion und Reinigung von Spülgut gelöst, welche die Merkmale des Hauptanspruches aufweist. Vorteilhafte Weiterbildungen der erfindungsgemäßen Vorrichtung sind in den abhängigen Unteransprüchen dargestellt.

Offenbart wird eine Vorrichtung zur Desinfektion und Reinigung von Spülgut, umfassend eine Kammer mit zumindest einem Sensor zur Erfassung von Prozessparametern, einen mit der Kammer verbundenen Ablauftrichter und eine Steuerung, die mindestens eine Steuereinheit, zur Steuerung eines Prozesses der Vorrichtung, mindestens eine Erfassungseinheit zur Erfassung der Werte des Sensors, und mindestens eine Recheneinheit zur Bestimmung der Hygienewirkung der Vorrichtung anhand des erfassten Wertes umfasst, wobei der Sensor im unteren Bereich der Kammer aufgenommen ist.

Im Sinne der vorliegenden Erfindung ist Spülgut Pflegegeschirr aus dem Bereich der Medizin, beispielsweise aus dem Bereich der Kranken- und Altenpflege oder medizinische Instrumente.

Um in der erfindungsgemäßen Vorrichtung vorgegebene Prozessparameter sicher und fehlerfrei erfassen zu können, umfasst die Vorrichtung zumindest einen Sensor. Es können auch mehr als ein Sensor oder mehrere Arten von Sensoren im unteren Bereich der Kammer der Vorrichtung aufgenommen sein. Weiterhin können Sensoren vorgesehen sein, die genau einen Prozessparameter erfassen, oder auch Sensoren, welche gleichzeitig oder zeitversetzt mehrere Arten von Prozessparametern erfassen können.

Im Sinne der vorliegenden Erfindung ist ein Prozessparameter ein zumindest teilweise den Reinigungs- und Desinfektionsprozess der Vorrichtung charakterisierender Parameter, der insbesondere Einfluss auf die Art und Weise der Reinigung und/oder Desinfektion (Hygiene) des Spülguts haben kann. Erfindungsgemäß ist ein Prozessparameter die Temperatur, insbesondere die Temperatur einer Flüssigkeit in der erfindungsgemäßen Vorrichtung, insbesondere in der Kammer.

Der zumindest eine Sensor ist erfindungsgemäß im unteren Bereich der Kammer, nämlich am tiefsten Punkt der Kammer, angeordnet. Die Kammer der Vorrichtung kann in zwei Bereiche aufgeteilt werden, nämlich in einen oberen Bereich und einen unteren Bereich. Im Sinne der vorliegenden Erfindung ist der obere Bereich als der Bereich definiert, in dem das Spülgut innerhalb der Kammer angeordnet ist. Der untere Bereich ist der Raum in der Kammer, der unterhalb des Spülguts definiert ist. Insbesondere umfasst der untere Bereich der Kammer den Bereich, in dem die Temperatur am Kühlsten ist. Somit umfasst der untere Bereich den kühlsten Punkt der Kammer, nämlich den Bereich in dem die Temperatur am Niedrigsten ist. Vorzugsweise ist der kühlste Punkt der Kammer auch gleichzeitig der tiefste Punkt der Kammer, welcher direkt über dem Ablauf, in den die Kammer mündet, angeordnet ist. Demnach liegt der kühlste Punkt in der Kammer im Pumpensumpf der Kammer. Der Pumpensumpf ist in der Kammer als der Bereich definiert, in dem sich das abzuführende Wasser sammelt, bevor es über den Ablauf in den Siphon geleitet wird.

Die Steuerung ist im Zusammenhang mit der vorliegenden Erfindung eine Vorrichtung, welche in der Lage ist, einen Reinigungsprozess und/oder einen Desinfektionsprozess in der Vorrichtung zu steuern und/oder zu regeln. Die Steuerung ist mehrteilig ausgestaltet und kann insbesondere eine oder mehrere Datenverarbeitungsvorrichtungen umfassen. Die Datenverarbeitungsvorrichtungen können einen oder mehrere Datenspeicher umfassen.

Die Steuerung umfasst mindestens eine Steuereinheit. Die Steuereinheit umfasst mindestens eine Datenverarbeitungsvorrichtung und/oder andere elektronische Vorrichtungen zum Steuern und/oder Regeln eines Prozesses der erfindungsgemäßen Vorrichtung.

Des Weiteren umfasst die Steuerung mindestens eine Erfassungseinheit. Im Sinne der vorliegenden Erfindung ist unter Erfassung, die Aufnahme der Werte des mindestens einen Sensors zu verstehen. Erfassung kann weiterhin auch eine Speicherung, beispielsweise eine Zwischenspeicherung, der Werte beinhalten. Die Erfassungseinheit verfügt über die entsprechenden Schnittstellen und Interfaces, um in Datenkommunikation mit dem Sensor und anderen elektronischen Bauteilen zu gehen.

Die Recheneinheit, im Sinne der Erfindung, umfasst zumindest ein Rechenmodul, welches in der Lage ist, mathematische Berechnungen in Echtzeit durchzuführen. Derartige geeignete Rechenmodule sind dem Fachmann bekannt. Diese Rechenmodule können Mikroprozessoren, Mikrochips oder Kleincomputer sein, wie diese zur Steuerung und Regelung von Haushalts-oder Industriegeräten verwendet werden. Die Rechnungseinheit verfügt über die entsprechenden Schnittstellen und Interfaces, um in Datenkommunikation mit der Erfassungseinheit und der Steuereinheit zu gehen.

Vorteilhaft an dieser Ausführungsform ist, dass der zumindest eine Sensor im unteren Bereich der Kammer angeordnet ist, wodurch realisierbar ist, dass der Sensor die Prozessparameter im kühlsten Bereich innerhalb der Kammer erfasst. Somit stellen die erfassten Prozessparameter keine verfälschten Werte dar, die aufgrund einer falschen Anordnung des Sensors innerhalb der Kammer, vorliegen würden. Somit kann gewährleistet werden, dass die auf das Spülgut einwirkende Hygienewirkung richtig erfasst werden kann.

Es ist klar, dass die Temperatur im unteren Bereich der Kammer in jedem Falle niedriger ist, als im Bereich der Kammer, wo sich das Spülgut befindet. Wird daher die niedrige Temperatur innerhalb der Kammer als Ausganspunkt für die Bestimmung der Hygienewirkung verwendet, so ist in jedem Falle sichergestellt, dass beispielsweise der geforderte und voreingestellte Ao-Wert erreicht wird.

Insbesondere bevorzugt ist eine erfindungsgemäße Vorrichtung, welche weiterhin einen Wassertank umfasst, welcher mit der Kammer verbunden ist und der mindestens einen weiteren Sensor aufweist. Der weitere Sensor ist auch derart ausgestaltet, um Prozessparameter zu erfassen, welche insbesondere die Temperatur einer Flüssigkeit innerhalb des Wassertanks sind. Der weitere Sensor ist vorzugsweise im unteren Bereich des Wassertanks, insbesondere unterhalb eines Wasserüberlaufs, welcher den Wassertank mit der Kammer verbindet, angeordnet. Vorteilhaft an dieser Anordnung ist, dass der weitere Sensor am kältesten Punkt des Wassertanks angeordnet ist und gewährleistet werden kann, dass die tatsächliche Temperatur des Wassers im Wassertank erfasst werden kann.

Bevorzugt ist eine erfindungsgemäße Vorrichtung, bei der die mindestens eine Recheneinheit derart eingerichtet ist, um entsprechend des Ergebnisses der Bestimmung der Hygienewirkung mindestens eine Information und/oder Anweisung an die mindestens eine Steuereinheit zu übermitteln.

Im Sinne der vorliegenden Erfindung können Informationen Angaben umfassen, ob ein Sollwert erreicht wurde oder Angaben über die aktuelle Abweichung des erfassten Wertes des mindestens einen Sensors verglichen mit dem vorgegebenen Sollwert. Anweisung, im Sinne der vorliegenden Erfindung, können Befehle an die Steuereinheit umfassen, wodurch Prozesse in der Vorrichtung geregelt oder gesteuert werden. Diese Anweisungen können beispielweise die Regelung der Zugabe von Kalt- oder Warmwasser oder von Dampf umfassen.

Vorteilhaft an dieser Ausführungsform ist, dass die Steuerung mittels des mindestens einen Sensors die erfindungsgemäße Vorrichtung überwacht. Die Überwachung kann in Intervallen erfolgen. Auf Grund der Überwachung können bei jeglichen Abweichungen von der geforderten Hygienewirkung geeignete Gegenmaßnahmen sofort und automatisch eingeleitet, durchgeführt und/oder überwacht werden.

Bevorzugt ist eine erfindungsgemäße Vorrichtung, bei der die mindestens eine Recheneinheit derart eingerichtet ist, um bei einer Übereinstimmung des erfassten Wertes des mindestens einen Sensors mit einem vorgegebenen Sollwert, eine Anweisung an die mindestens eine Steuereinheit zu übermitteln, insbesondere eine den Desinfektions- oder Reinigungsprozess beeinflussende.

Vorteilhaft an dieser Ausführungsform ist, dass bei Erreichen des vorgegebenen Sollwertes, zum Beispiel über ein optisches oder akustisches Signal, das Ende des Desinfektions- und/oder Reinigungsprozesses angezeigt werden kann. Somit ist eine unverzügliche Beendigung des Prozesses möglich und dadurch unterbleibt eine unerwünschte Laufzeitverlängerung des Desinfektions- und/oder Reinigungsprozesses. Dies schont die Ressourcen und setzt darüber hinaus den Energieverbrauch der Vorrichtung während des Betriebes bei jedem Reinigungs- und/oder Desinfektionsprozess erheblich herab, wodurch eine Kosteneinsparung erfolgt.

Offenbart wird eine Vorrichtung, bei der der vorgegebene Sollwert den A0-Werten der EN DIN ISO 15883-1 sowie der EN DIN ISO 15883-3 entspricht.

In der erfindungsgemäßen Vorrichtung ist der mindestens eine Sensor ein Temperatursensor.

Bevorzugt ist außerdem eine erfindungsgemäße Vorrichtung, bei welcher der Temperatursensor ausgewählt ist aus Pt 100-, Pt 500- oder Pt 1000-Sensoren, Heißleitern, NTC-Widerständen oder NTC-Thermistoren. Besonders bevorzugt sind Pt 100-, Pt 500- und Pt 1000-Sensoren. Dies sind Platin-Messwiderstände, die als Messeffekt die Abhängigkeit des elektrischen Widerstands von der Temperatur bei Platin anwenden. Sie sind in der EN 60751 genormt. Durch ihre geringen Grenzabweichungen sind diese in aller Regel ohne Neukalibrierung austauschbar.

Die Normung umfasst im Allgemeinen den Bereich -200 °C bis 850 °C, der tatsächliche Einsatzbereich eines Platin-Messwiderstands ist jedoch meistens enger begrenzt und im Datenblatt spezifiziert.

Platin-Messwiderstände werden nach ihrem Material und ihrem Nennwiderstand bei einer Temperatur von 0 °C bezeichnet. Hierbei hat Pt 100 einen Nennwiderstand von 100 Ω, Pt 500 von 500 Ω und Pt 1000 von 1 kΩ.

Besonders bevorzugt ist eine erfindungsgemäße Vorrichtung, bei der die mindestens eine Steuerung, insbesondere die mindestens eine Recheneinheit mindestens eine Speichereinheit umfasst.

Im Sinne der vorliegenden Erfindung kann die Speichereinheit separat von der Recheneinheit oder auch ganz oder teilweise als Teil der Recheneinheit ausgestaltet sein. Vorzugsweise ist die Speichereinheit unabhängig von der Steuereinheit ausgebildet, wobei jedoch alternativ auch eine vollständige oder teilweise Integration in diese Steuereinheit möglich ist. Die Recheneinheit soll vorzugsweise derart eingerichtet sein, um Daten und Ereignisse zu speichern, vorzugsweise unabhängig von der Steuereinheit.

Insbesondere bevorzugt ist eine erfindungsgemäße Vorrichtung, bei der die mindestens eine Recheneinheit derart eingerichtet ist, um auf die mindestens eine Speichereinheit zuzugreifen und den erfassten Wert des mindestens einen Sensors sowie die ermittelte Hygienewirkung in den Datenspeicher der mindestens einen Speichereinheit zu implementieren.

Weiterhin bevorzugt ist eine erfindungsgemäße Vorrichtung, bei der die mindestens eine Recheneinheit derart eingerichtet ist, dass sie mit der mindestens einen Steuereinheit über eine kabellose Verbindung kommunizieren kann.

Eine kabellose Verbindung kann im Sinne der vorliegenden Erfindung ausgewählt sein aus WLAN, Bluetooth oder Mobilfunk.

Vorteilhaft an dieser Ausführungsform ist, dass die Recheneinheit und die Steuereinheit getrennt beziehungsweise separat voneinander angeordnet sein können.

Offenbart wird ferner ein Verfahren zur Reinigung und Desinfektion von Spülgut mittels einer erfindungsgemäßen Vorrichtung, wobei man über den mindestens einen Sensor und die mindestens eine Steuerung, die auf das Spülgut einwirkende Temperatur erfasst und man daraus die entsprechende Hygienewirkung bestimmt.

Vorteilhaft an dieser Ausführungsform ist, dass gewährleistet werden kann, dass die notwendige Hygienewirkung auf das Spülgut wirkt. Gegebenenfalls kann der Reinigungs- und/oder Desinfektionsprozess in der Vorrichtung gesteuert und/ oder geregelt werden, wenn die vorgegebene Hygienewirkung noch nicht erreicht ist, da diese vom vorgegebenen Sollwert abweicht. Diese Steuerung und/oder Regelung kann sowohl manuell als auch automatisch erfolgen.

Es ist erfindungsgemäß auch vorgesehen, mehrere Vorrichtungen mittels Datenverbindungen miteinander zu vernetzen und/oder an eine zentrale Recheneinheit anzuschließen. Dadurch ist es möglich, die einzelnen Desinfektions- und Reinigungsvorgänge in den jeweiligen Vorrichtungen zentral zu erfassen und zu überwachen und dabei auch zu protokollieren, um den Anforderungen der EN DIN ISO 15883 zu genügen. Diese Vernetzung der einzelnen Geräte ist insbesondere in größeren Krankenhäusern oder Pflegeeinrichtungen sinnvoll, da die Einhaltung der Hygienevorschriften kritisch sein kann. Die vorliegende Erfindung wird mit den beigefügten Zeichnungen näher erläutert. Es zeigt:

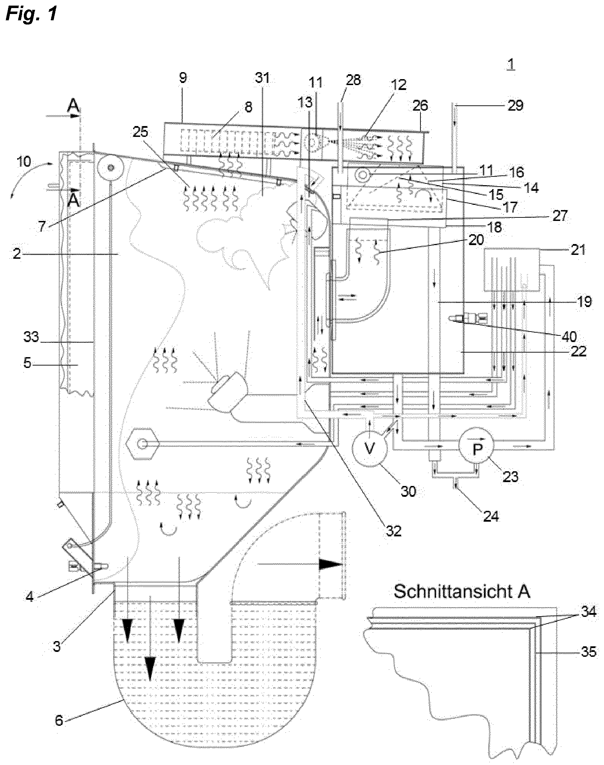

Fig. 1 ein Ausführungsbeispiel einer erfindungsgemäßen Vorrichtung.

Die vorliegende Erfindung offenbart eine Vorrichtung zur Desinfektion und Reinigung von Spülgut. Diese Vorrichtung kann dazu verwendet werden, die volle Hygienewirkung auf dem Spülgut zu gewährleisten.

In der erfindungsgemäßen Vorrichtung wird ein Temperatursensor, am kältesten Punkt innerhalb der Kammer der Vorrichtung angeordnet. Da die Wärme innerhalb der Kammer nach oben steigt, ist der kälteste Punkt in der Kammer im unteren Bereich angeordnet. Eine besonders vorteilhafte Anordnung des Sensors ist im Pumpensumpf der Kammer.

Vorteilhaft an der Anordnung des Sensors am kältesten Punkt der Kammer ist, dass dadurch gewährleistet wird, dass im gesamten Spülbereich nicht nur in der Waschkammer die entsprechend benötigte Temperatur erreicht wird, um die geforderte Hygienewirkung zu gewährleisten.

Die nachfolgend beschriebenen Ausführungsformen erläutern die Erfindung näher, ohne den Umfang der Erfindung zu beschränken.

Die Figur 1 zeigt eine erfindungsgemäße Vorrichtung 1, wie sie beispielsweise in einem Krankenhaus zum Reinigen und Desinfizieren von medizinischen Spülgütern verwendet wird. Hierzu umfasst die Vorrichtung 1 eine Kammer 2 für die Aufbereitung des Spülguts, die in einen Ablauftrichter 3 mündet. Der Ablauftrichter 3 ist üblicherweise mit einem Siphonbogen 6 für den Ablauf versehen, um das verbrauchte Waser und jegliche Verschmutzungen in das Abwassersystem zu leiten.

Die Kammer 2 umfasst einen Sensor 4, der im unteren Bereich aufgenommen ist. Der untere Bereich der Kammer 2 ist der kälteste Bereich der Kammer 2 und besonders vorteilhaft ist die Anordnung des Sensors 4 im Pumpensumpf oder in dessen unmittelbarer Nähe. Die Anordnung wird aber auch derart gewählt, dass eine Verschmutzung durch die Anhaftungen am Spülgut vermieden wird, wenn diese in den Pumpensumpf geleitet werden.

Der Sensor 4 ist ein Temperatursensor. Durch die Anordnung des Temperatursensors am kältesten Punkt der Kammer 2, kann gewährleistet werden, dass die volle Desinfektionswirkung auf das Spülgut wirkt. Insbesondere ist die Anordnung des Temperatursensors im Pumpensumpf vorteilhaft.

Der Sensor 4 übermittelt die gemessenen Werte, insbesondere die gemessene Temperatur, an eine Steuerung (nicht dargestellt) der Vorrichtung. Die Steuerung umfasst eine Steuereinheit, eine Erfassungseinheit und eine Recheneinheit. Die Erfassungseinheit erfasst die vom Sensor 4 übermittelten Werte und leitet diese an die Recheneinheit weiter. Die Recheneinheit der Steuerung vergleicht die vom Sensor 4 ermittelten Werte mit Sollwerten, die in einem Datenspeicher abgelegt sind. Die Sollwerte entsprechen den Ao-Werten nach der EN DIN ISO 15833-1.

Die Recheneinheit ist derart eingerichtet, dass sie im Falle, dass der erfasste Wert des Sensors 4 mit einem Sollwert übereinstimmt, der Steuereinheit eine Anweisung übermittelt, die den Desinfektions- und Reinigungsprozess beeinflusst. Eine solche Anweisung kann die Beendigung des Desinfektions- und Reinigungsprozesses beinhalten. Sollte der Sollwert noch nicht erreicht sein, so kann die Recheneinheit der Steuereinheit eine Information und/oder Anweisung übermitteln, wobei hierbei die Anweisung sein kann, dass beispielweise die Zugabe von Kalt- oder Warmwasser oder Dampf geregelt werden soll.

Somit wird durch die Steuerung über den Sensor 4 die erfindungsgemäße Vorrichtung 1 überwacht. Die Überwachung kann in Intervallen erfolgen. Auf Grund der Überwachung können bei jeglichen Abweichungen von der geforderten Hygienewirkung geeignete Gegenmaßnahmen sofort und automatisch eingeleitet, durchgeführt und/oder überwacht werden.

Einzelne Komponenten der Steuerung kommunizieren über Kabelverbindung oder kabellose Verbindung miteinander. Die Recheneinheit der Steuerung ist derart eingerichtet, dass sie über kabellose Verbindung bzw. drahtlos mit der Steuereinheit kommunizieren kann.

Des Weiteren umfasst die erfindungsgemäße Vorrichtung 1 eine Tür 5, mit der die Kammer 2 verschlossen werden kann. Die Tür 5 sowie die Kammer 2 weisen ein Labyrinth 33 als Dichtung anstatt einer Türdichtung auf.

Ein Wassertank 22 zur Aufbereitung von Kaltwasser und/oder Warmwasser 29 mit der Möglichkeit der Zugabe von Mitteln 28 ist über einen Wasserüberlauf 27 mit der Kammer 2 verbunden. In dem Wassertank 22 ist ein weiterer Sensor 40 angeordnet. Der weitere Sensor 40 ist vorzugsweise im unteren Bereich des Wassertanks 22 angeordnet. Der weitere Sensor 40 ist ein Temperatursensor oder Feuchtigkeitssensor, insbesondere bevorzugt ist ein Temperatursensor. Durch die Anordnung des Temperatursensors am kältesten Punkt des Wassertanks 22, kann gewährleistet werden, dass die tatsächliche Temperatur des Wassers im Wassertank 22 erfasst wird.

Auch der weitere Sensor 40 ist derart eingerichtet, dass dieser die gemessenen Werte, insbesondere die gemessene Temperatur, an die Steuerung (nicht dargestellt) der Vorrichtung 1 übermittelt. Die Erfassungseinheit erfasst die vom weiteren Sensor 40 übermittelten Werte und leitet diese an die Recheneinheit weiter. Die Recheneinheit der Steuerung vergleicht die vom weiteren Sensor 40 ermittelten Werte mit Sollwerten, die in einem Datenspeicher abgelegt sind und entsprechend eine Information und/oder Anweisung an die Steuereinheit übermittelt.

Die Mittel, welche in den Wassertank 22 hinzugegeben werden können, umfassen beispielsweise Desinfektionsmittel, Reinigungsmittel oder Wasseraufbereitungsmittel, wie Entkalkungsmittel oder Tenside. Für die thermische Desinfektion in der Vorrichtung 1 wird ein Verdampfer 30 verwendet, der außerhalb der Kammer 2 der Vorrichtung 1angeordnet ist. Der Verdampfer 30 ist über eine Dampfleitung 32 mit der Kammer 2 verbunden, worüber der Wasserdampf 31 in die Kammer 2 eingebracht wird. Nach der thermischen Desinfektion kann eine Rückkühlung durchgeführt werden, wobei das Dampf/Luftgemisch 25 aus der Kammer 2 in Intervallen abgesaugt wird und über einen Kondensator 17, der außerhalb der Kammer 2 angeordnet ist, geleitet wird und als entfeuchtete und rückgekühlte Luft in die Kammer 2 zurückgeführt wird. Dadurch wird ein geschlossener Kreislauf geschaffen, der verhindert, dass von außen nach innen und/oder von innen nach außen jegliche Gase, Flüssigkeiten ein- bzw. ausdringen können.

Außerdem weist die erfindungsgemäße Vorrichtung 1 einen Lüfter 8 auf. Im Sinne der vorliegenden Erfindung kann der Lüfter 8 auch ein Gebläse sein. Der Lüfter kann jeder dem Fachmann bekannten Art oder Bauart entsprechen, um das Dampf/Luftgemisch 25 aus der Kammer 2 abzusaugen. Der Lüfter 8 ist indirekt oder direkt mit der Kammer 2 verbunden. In der Kammer 2, ist unterhalb des Lüfters 8 ein Prallblech 7 angeordnet. Das Prallblech 7 ist über der Ansaugöffnung angeordnet, welche aus einer oder mehreren definierten Durchlassöffnungen besteht.

Der Lüfter 8 ist in einem Lüftergehäuse 9 angeordnet, welches aus der Aufnahme für den Lüfter, dem Luftkanal 26 und mindestens einer Kondensatdüse 11 besteht. Die Kondensatdüse 11 kann während des Rückkühlens und der thermischen Desinfektion einen feinen Wassernebel 12 aus Kaltwasser in den Luftkanal 26 einbringen.

Das Lüftergehäuse 9 ist mit einem Kondensator 17 verbunden, der innerhalb des Wassertanks 22 angeordnet ist. Der Kondensator 17 umfasst einen vom Wassertank 22 getrennten Kondensatablauf 19. Daher kommt der Wrasen nicht in Verbindung mit dem Spülwasser. Der Kondensator 17 besteht aus zwei Teilen, dem Kondensatorboden 18, der den Kondensatablauf 19 aufweist und dem Kondensatordeckel 14. Der Kondensatordeckel 14 umfasst eine Hutze 16, ein Leitblech 15 und mindestens eine Kondensatdüse 11. Der Kondensator 17 ist mit einer Kammerentlüftung 20 verbunden. Die Kammerentlüftung 20 dient als Wasserüberlauf 27 des Wassertanks 22, als Strömungsöffnung und als Luftrückführung in die Kammer 2.

Die mindestens eine Kondensatdüse 11 sprüht einen feinen, aus Kaltwasser bestehenden, Wassernebel 12 auf die Hutze 16 und kühlt diese somit von außen ab. Durch die Abkühlung schlägt sich der Wasserdampf 31, der aus der Kammer 2 abgesaugt wird und in den Kondensator 17 eingeleitet wird, im Innern der Hutze 16 als Kondensat nieder und kühlt ab. Mittels der Kammerentlüftung 20 wird die abgekühlte und entfeuchtete Luft in die Kammer zurückgeführt. Der Kondensatablauf 19 ist im Kondensatorboden 18 angeordnet, um das Kondensat wegzuführen. Somit wird in der erfindungsgemäßen Vorrichtung 1 eine Rückverkeimung des desinfizierten Spülguts ausgeschlossen.

### Bezugszeichenliste

- 1: Vorrichtung
- 2: Kammer
- 3: Ablauftrichter
- 4: Sensor
- 40: weiterer Sensor
- 5: Tür
- 6: Siphon
- 7: Prallblech
- 8: Lüfter
- 9: Lüftergehäuse
- 10: Schließ- und Öffnungsrichtung
- 11: Kondensatdüse
- 12: Wassernebel
- 13: Düse
- 14: Kondensatordeckel
- 15: Leitblech
- 16: Hutze
- 17: Kondensator
- 18: Kondensatorboden
- 19: Kondensatablauf
- 20: Kammerentlüftung
- 21: Verteiler
- 22: Wassertank
- 23: Pumpe
- 24: Restentleerung
- 25: Dampf/Luftgemisch
- 26: Luftkanal
- 27: Wasserüberlauf
- 28: Zulauf Wasserkonditonierer/Mittel
- 29: Kaltwasser und/oder Warmwasser bzw. Medium
- 30: Verdampfer
- 31: Wasserdampf
- 32: Dampfleitung
- 33: Labyrinthdichtung
- 34: Labyrinthblech der Kammer
- 35: Labyrinthblech der Tür

## Patentansprüche

1. Vorrichtung (1) zur thermischen Desinfektion mittels Wasserdampf und zur Reinigung von medizinischem Spülgut, umfassend eine Kammer (2), einen Verdampfer (30), der über eine Dampfleitung (32) mit der Kammer (2) verbunden ist, worüber Wasserdampf (31) in die Kammer (2) einbringbar ist, einen Ablauftrichter (3), in den die Kammer (2) mündet und der mit einem Siphonbogen (6) versehen ist, wobei der Siphonbogen (6) vorgesehen ist, um verbrauchtes Wasser und Verschmutzungen in das Abwassersystem zu leiten, zumindest einem Sensor (4) zur Erfassung von Prozessparametern,
und eine Steuerung, die mindestens eine Steuereinheit zur Steuerung eines Prozesses der Vorrichtung (1), mindestens eine Erfassungseinheit zur Erfassung der Werte des mindestens einen Sensors (4) zur Erfassung von Prozessparametern und mindestens eine Recheneinheit zur Bestimmung der Hygienewirkung der Vorrichtung (1) anhand des erfassten Wertes, umfasst,
**dadurch gekennzeichnet, dass** der mindestens eine Sensor (4) zur Erfassung von Prozessparametern ein Temperatursensor ist und im tiefsten Punkt der Kammer (2) angeordnet ist.

2. Vorrichtung (1), gemäß Anspruch 1, **dadurch gekennzeichnet, dass** die Vorrichtung weiterhin einen Wassertank (22) umfasst, welcher mit der Kammer (2) verbunden ist und der mindestens einen weiteren Sensor (40) aufweist.

3. Vorrichtung (1), gemäß Anspruch 1 oder 2, **dadurch gekennzeichnet, dass** die mindestens eine Recheneinheit derart eingerichtet ist, um entsprechend des Ergebnisses der Bestimmung der Hygienewirkung mindestens eine Information und/oder Anweisung an die mindestens eine Steuereinheit zu übermitteln.

4. Vorrichtung (1), gemäß einem der Ansprüche 1 bis 3, **dadurch gekennzeichnet, dass** die mindestens eine Recheneinheit derart eingerichtet ist, um bei einer Übereinstimmung des erfassten Wertes des mindestens einen Sensors (4, 40) mit einem vorgegebenen Sollwert, eine Anweisung an die mindestens eine Steuereinheit zu übermitteln, insbesondere eine den Desinfektions- oder Reinigungsprozess beeinflussende.

5. Vorrichtung (1), gemäß Anspruch 1, **dadurch gekennzeichnet, dass** der Temperatursensor ausgewählt ist aus Pt 100-, Pt 500- oder Pt 1000-Sensoren, Heißleitern, NTC-Widerständen oder NTC-Thermistoren.

6. Vorrichtung (1), gemäß einem der vorhergehenden Ansprüche, **dadurch gekennzeichnet, dass** die mindestens eine Steuerung, insbesondere die mindestens eine Recheneinheit, mindestens eine Speichereinheit umfasst.

7. Vorrichtung (1), gemäß Anspruch 68, **dadurch gekennzeichnet, dass** die mindestens eine Recheneinheit derart eingerichtet ist, um auf die mindestens eine Speichereinheit zuzugreifen und den erfassten Wert des mindestens einen Sensors sowie die ermittelte Hygienewirkung in den mindestens einen Datenspeicher zu implementieren.

8. Vorrichtung (1), gemäß einem der vorhergehenden Ansprüche, **dadurch gekennzeichnet, dass** die mindestens eine Recheneinheit derart eingerichtet ist, dass sie mit der mindestens einen Steuereinheit über eine kabellose Verbindung kommunizieren kann.

## Claims

1. Device (1) for thermal disinfection by means of steam and for cleaning medical wash ware, comprising a chamber (2), an evaporator (30) which is connected to the chamber (2) via a steam line (32), via which steam (31) can be introduced into the chamber (2), a discharge funnel (3) into which the chamber (2) opens and which is provided with a siphon bend (6), the siphon bend (6) being provided for discharging used water and contaminants into the waste water system, at least one sensor (4) for detecting process parameters
and a control system comprising at least one control unit for controlling a process of the device (1), at least one detection unit for detecting the values of the at least one sensor (4) for detecting process parameters, and at least one calculation unit for determining the hygienic effect of the device (1) on the basis of the detected value,
**characterised in that** the at least one sensor (4) for detecting process parameters is a temperature sensor and is arranged in the lowest point of the chamber (2).

2. Device (1) according to claim 1, **characterised in that** the device further comprises a water tank (22) which is connected to the chamber (2) and which has at least one further sensor (40).

3. Device (1) according to claim 1 or 2, **characterised in that** the at least one calculation unit is set up to transmit at least one information and/or instruction to the at least one control unit according to the result of the determination of the hygienic effect.

4. Device (1) according to one of claims 1 to 3, **characterised in that** the at least one calculation unit is set up in such a way that, if the detected value of the at least one sensor (4, 40) corresponds to a predetermined setpoint value, an instruction is transmitted to the at least one control unit, in particular an instruction which influences the disinfection or cleaning process.

5. Device (1) according to claim 1, **characterised in that** the temperature sensor is selected from Pt 100, Pt 500 or Pt 1000 sensors, thermistors, NTC resistors or NTC thermistors.

6. Device (1) according to one of the preceding claims, **characterised in that** the at least one control unit, in particular the at least one calculation unit, comprises at least one memory unit.

7. Device (1) according to claim 6, **characterised in that** the at least one calculation unit is set up to access the at least one memory unit and to implement the detected value of the at least one sensor and the determined hygiene effect in the at least one data memory.

8. Device (1) according to any one of the preceding claims, **characterised in that** the at least one calculation unit is arranged to communicate with the at least one control unit via a wireless connection.

## Revendications

1. Dispositif (1) pour la désinfection thermique au moyen de vapeur d'eau et pour le nettoyage d'articles médicaux à laver, comprenant une chambre (2), un évaporateur (30), qui est relié à la chambre (2) par une conduite de vapeur (32), par laquelle de la vapeur d'eau (31) est apte à être introduite dans la chambre (2), un entonnoir d'évacuation (3) dans lequel débouche la chambre (2) et qui est pourvu d'un coude de siphon (6), le coude de siphon (6) étant prévu pour évacuer l'eau usée et les impuretés dans le système d'eaux usées, au moins un capteur (4) pour détecter des paramètres de processus,
et une commande qui comprend au moins une unité de commande pour commander un processus du dispositif (1), au moins une unité de détection pour détecter les valeurs dudit au moins un capteur (4) pour détecter des paramètres de processus et au moins une unité de calcul pour déterminer l'effet hygiénique du dispositif (1) à l'aide de la valeur détectée,
**caractérisé en ce que** ledit au moins un capteur (4) pour la détection de paramètres de processus est un capteur de température et est disposé au point le plus bas de la chambre (2).

2. Dispositif (1) selon la revendication 1, **caractérisé en ce que** le dispositif comprend en outre un réservoir d'eau (22) qui est relié à la chambre (2) et qui comporte au moins un autre capteur (40).

3. Dispositif (1) selon la revendication 1 ou la revendication 2, **caractérisé en ce que** ladite au moins une unité de calcul est agencée de manière à transmettre au moins une information et/ou une instruction à ladite au moins une unité de commande en fonction du résultat de la détermination de l'effet hygiénique.

4. Dispositif (1) selon l'une des revendications 1 à 3, **caractérisé en ce que** ladite au moins une unité de calcul est agencée de manière à transmettre une instruction à ladite au moins une unité de commande, en particulier une instruction influençant le processus de désinfection ou de nettoyage, en cas de concordance de la valeur détectée par ledit au moins un capteur (4, 40) avec une valeur de consigne prédéfinie.

5. Dispositif (1), selon la revendication 1, **caractérisé en ce que** le capteur de température est choisi parmi les capteurs Pt 100, Pt 500 ou Pt 1000, les thermistances, les résistances NTC ou les thermistances NTC.

6. Dispositif (1), selon l'une quelconque des revendications précédentes, **caractérisé en ce que** ladite au moins une commande, notamment ladite au moins une unité de calcul, comprend au moins une unité de mémoire.

7. Dispositif (1) selon la revendication 6, **caractérisé en ce que** ladite au moins une unité de calcul est agencée de manière à accéder à ladite au moins une unité de mémoire et à mettre en œuvre la valeur détectée dudit au moins un capteur ainsi que l'effet hygiénique déterminé dans ladite au moins une mémoire de données.

8. Dispositif (1) selon l'une quelconque des revendications précédentes, **caractérisé en ce que** ladite au moins une unité de calcul est agencée de manière à être apte à communiquer avec ladite au moins une unité de commande par une connexion sans fil.
